# EUROPÄISCHE PATENTSCHRIFT

(11) **EP 0 594 943 B1**
(45) Veröffentlichungstag und Bekanntmachung des Hinweises auf die Patenterteilung: **30.09.1998**
(21) Anmeldenummer: 93109409.8
(22) Anmeldetag: 11.06.1993
(51) Int. Cl.: A61K 33/42, A61K 9/20, A61K 9/28

(54) **Pharmazeutische Zubereitung für die Fluoridionen-Versorgung**
Pharmazeutical preparation for the administation of fluoride ions
Préparation pharmaceutique pour l'administration d'ions fluors

(30) Priorität: 26.10.1992 DE 4236090
(43) Veröffentlichungstag der Anmeldung: 04.05.1994
(73) Patentinhaber: ASTA MEDICA ARZNEIMITTEL Ges.m.b.H., 1230 Wien (AT)
(72) Erfinder: Tritthart, Wolfram, Dr., A-9400 Wolfsberg (AT); Wolf, Rüdiger, Dr., A-2380 Perchtollsdorf (AT)
(74) Vertreter: Winkler, Andreas, Dr.

(56) Entgegenhaltungen:
- EP-A- 0 446 753
- EP-A- 0 503 425
- GB-A- 2 195 245
- US-A- 5 039 526

## Beschreibung

Die Erfindung betrifft eine pharmazeutische Zubereitung für die Fluoridionen-Versorgung zur Behandlung und Verhinderung von Knochensubstanzverlusten, einschließlich Osteoporosis und alveolaren Knochenschädigungen, mit einem Gehalt an Natriumfluorophosphat und Mitteln zur Regulierung der Natriumfluorophosphat-Freigabe.

Es ist bekannt, daß Fluoride die Aktivität von knochenbildenden Zellen stimulieren und zusammen mit Calcium und Phosphat, den Hauptbestandteilen des Knochens, in der Knochenstruktur gespeichert werden.

Aus der US-PS 32 87 219 ist bekannt, daß durch orale Verabreichung von Natriumfluorid die Knochenheilung verbessert werden kann.

Darüber hinaus wurde in letzter Zeit zunehmend der Einsatz von Natriumfluorid oder Natriumfluorophosphat zur Behandlung von Knochenschäden diskutiert. So wird in Europa bspw. unter der Bezeichnung "Flurexal®" eine Tablette mit einem Gehalt von 22 mg Natriumfluorid entsprechend 10 mg F von der Firma Zyma S.A., Nyon, Schweiz, oder unter der Bezeichnung "Tridin®" eine Kautablette mit einem Gehalt von 38 mg Natriumfluorophosphat entsprechend 5 mg F von der Opfermann Arzneimittel GmbH vertrieben. "Flurexal®" soll dreimal täglich, "Tridin®" in einer Menge von 1 bis 2 Tabletten dreimal täglich zur Behandlung von Steroid-Osteoporosis oder mit einer Tablette dreimal täglich zur Verhinderung dieser verabreicht werden.

Die Verbraucherinformation zu "Tridin®" erwähnt, daß gastrische und intestinale Reizungen nur selten beobachtet werden. In Caries Res. 17 (Suppl. 1), Seiten 46 bis 55 (1983) wird von Yngve Ericsson in "Monofluorophosphate Physiology: General Considerations" berichtet, daß weder bei Patienten noch in zahlreichen Untersuchungen mit Laboratoriumsangestellten eine subjektive Beeinträchtigung bei Dosen bis zu 30 mg F als Fluorophosphat beobachtet wurde. Zwischenzeitlich durchgeführte klinische Untersuchungen und Patientenberichte zeigen jedoch, daß in einer beachtlichen Zahl von Fällen gastrische und intestinale Störungen auftraten.

Zur Behebung dieser Nachteile sind in den DE-OSen 37 27 615 und 37 27 616 gattungsgemäße pharmazeutische Zubereitungen beschrieben (im Falle der DE-OS 37 27 615 mit einem zusätzlichen Gehalt an einer calciumhaltigen Zusammensetzung), bei denen Mittel zur Natriumfluorophosphat-Freigabe in Form von in Wasser quellbarem Cellulosepulver oder Cellulosefäden vorgeschlagen werden, die ein zusammenhängendes Netzwerk als Matrix bilden, in der das Natriumfluorophosphat gleichmäßig und homogen dispergiert ist, wobei nach Zugabe der einheitlichen Verabreichungsform ein wäßriges Medium das Cellulosepulver oder die Cellulosefäden an der Oberfläche der Verabreichungsform weich werden und sich von dem restlichen Material lösen und damit einen Teil des Natriumfluorophosphats freisetzen. Durch diese Matrix soll erreicht werden, daß das Fluor nur verzögert oder sehr langsam innerhalb der ersten 1 bis 8 Stunden freigegeben wird, so daß die Verabreichungsform durch den Magen hindurchgeht und in den Intestinaltrakt gelangt, bevor die gleichmäßige Freigabe erfolgt.

Genauere Untersuchungen haben allerdings ergeben, daß die Wirkstoffreisetzung nicht linear erfolgt. Nach 1 h sind bereits 40 % Wirkstoff freigesetzt, nach 5 h 70 %, nach 6 h wird ein Plateau von 75 % erreicht. Eine Freisetzungsprüfung in künstlichen Magensaft ergab nach 2 h bereits 2 % freies Fluorid, bezogen auf die gesamte Verabreichungsform, was bezogen auf Gesamtfluorid, etwa 15,2 % entspricht, einem Wert, der einer Freisetzung von Natriumfluorophosphat ohne Retardsystem weitgehend entspricht. Dies läßt eine erhöhte Nebenwirkungsrate erwarten.

In-vivo-Daten belegen diese in-vitro-Untersuchungen. Bei Daten von sechs Patienten konnte nachgewiesen werden, daß nach 3 h bereits ein Peak-Maximum erreicht wird. Das therapeutische Ziel der bekannten Zubereitung einer kontrollierten Wirkstofffreisetzung von bis zu 8 h in vivo wird nicht erreicht. Auch erfolgt die Freisetzung des Fluors bereits zu mehr als 50 % innerhalb der ersten Stunde.

Der vorliegenden Erfindung liegt daher die Aufgabe zugrunde, daß bekannte Retardsystem dahingehend weiterzubilden, daß die Freisetzung möglichst linear und mindestens über den bei der bekannten Zubereitung angegebenen, aber von dieser nicht erreichten Zeitraum von mehr als 8 Stunden erfolgt.

Erfindungsgemäß wird diese Aufgabe dadurch erreicht, daß die Mittel zur Regulierung der Natriumfluorophosphat-Freigabe ein duales Retardsystem umfassen, das aus einer Retardmatrix im Tablettenkern und einem retardierenden Überzug besteht.

Dabei ist es bevorzugt, daß die Retardmatrix auf Copolymerisaten aus Acryl- und Methacrylsäureestern aufgebaut ist.

Die Retardmatrix ist vorzugsweiseauf einer wäßrigen Dispersion von Copolymerisaten aus Acryl- und Methacrylsäureestern mit einem geringen Gehalt an quartären Ammoniumgruppen aufgebaut.

Besonders bevorzugt ist die Retardmatrix auf einem Gemisch von Eudragit® RSPM und Eudragit® RS30D aufgebaut, wobei Eudragit® RSPM und Eudragit® RS30D vorzugsweisein einem Verhältnis von etwa 4:1 bis 1:1 vorliegen.

Die Erfindung schlägt vor, daß der retardierende Überzug auf Copolymerisaten aus Acryl- und Methacrylsäureestern aufgebaut ist.

Vorzugsweise ist der retardierende Überzug auf einer wäßrigen Dispersion von Copolymerisaten aus Acryl- und Methacrylsäureestern mit einem geringen Gehalt an quartären Ammoniumgruppen aufgebaut, wobei besonders bevorzugt der retardierende Überzug auf Eudragit® RS30D aufgebaut ist.

Folgende Zusammensetzung gelöst:

| | | |
|---|---|---|
| Kern: | Natriumfluorophosphat | 79,80 mg |
| | Eudragit® RSPM | 63,55 mg |
| | Eudragit® RS 30 D | 25,50 mg |
| | Magnesiumstearat | 3,00 mg |
| | Boeson® VP | 3,00 mg |
| | Stearinsäure-Pulver | 4,00 mg |
| | Aerosil 200 | 1,00 mg |
| Überzug: | Talk | 2,90 mg |
| | Titandioxid | 2,05 mg |
| | Diethylphthalat | 0,34 mg |
| | Eudragit® RS 30 D | 5,70 mg |

Erfindungsgemäß ist ein Gehalt von 120 bis 200 mg Natriumfluorophosphat bei einer einmal täglich zu applizierenden Formulierung bzw. ein Gehalt von 40 bis 100 mg Natriumfluorophosphat bei einer zweimal täglich zu applizierenden Formulierung vorgesehen.

Mit der vorliegenden Erfindung ist es erstmals gelungen, die Nachteile des Standes der Technik wirkungsvoll zu beseitigen. Erreicht wurde dies durch das neuartige duale Retardsystem, das im Kern bevorzugterweise aus Copolymerisaten aus Acryl- und Methacrylsäureestern besteht. Bei diesen Materialien ist zusätzlich von besonderem Interesse, daß sie eine Herstellung ohne organische Lösungsmittel erlauben, was angesichts der üblichen Retardierungsmittel, nämlich in organischen Lösungsmitteln gelösten Acryllackharzsubstanzen, einen bedeutenden Vorteil darstellt.

Zusätzliche Bestandteile im Kern können Aerosil als Fließmittel und Magnesiumstearat, Boeson VP® und Stearinsäure als Gleit- und Formtrennmittel sein.

Ein weiteres wesentliches Element - neben dem speziellen Charakter der Retardmatrix - der vorliegenden Erfindung gegenüber der DE-OS 37 27 616 ist das Vorsehen eines zusätzlichen retardierenden Überzugs, der ebenfalss bevorzugt auf der Grundlage von Copolymerisaten aus Acryl- und Methacrylsäureestern aufgebaut ist; und zusätzlich als Gleitmittel Talk, als Farbstoff Titandioxid und als Weichmacher Diethylphtalat enthalten kann.

Die Kombination von erfindungsgemäßer Retardmatrix und als Diffusionsmembrane ausgebildetem Überzug führt zu einer nicht vorhersehbaren überadditiven Hemmung der Wirkstofffreisetzung von Natriumfluorophosphat und ermöglicht daher erstmals das Erreichen einer Wirkstofffreisetzung und eines Wirkstoffplateaus in den angestrebten Zeiträumen.

Überraschenderweise hat sich ergeben, daß die neuartige pharmazeutische Zubereitung die gewünschte Wirkung im Hinblick auf die Fluoridfreisetzung zur Verfügung stellen kann. Dabei erfolgt die Wirkstofffreisetzung bei der einmal täglich zu applizierenden Formulierung in einer Zeitspanne von 8,5 bis 12 Stunden, während sie bei der zweimal täglich zu applizierenden Formulierung in einem Zeitraum von 5 bis 8 Stunden erfolgt. Es hat sich herausgestellt, daß im künstlichen Magensaft nach 2 Stunden weniger als 1 % freies Fluorid - bezogen auf Gesamtnatriumfluorophosphat - freigesetzt sind, so daß gastrointestinale Nebenwirkungen wirkungsvoll verhindert werden. Die neuartige pharmazeutische Zubereitung erreicht ein Wirkstoffplateau in vivo von 10 bis 18 Stunden bei der einmal täglich zu applizierenden Formulierung und von 6 bis 12 Stunden bei der zweimal täglich zu applizierenden Formulierung.

Die in einer besonderen Ausführungsform der erfindungsgemäßen Zubereitung durchgeführten in-vitro- und in-vivo-Untersuchungen werden im Detail an den beigefügten Zeichnungen näher erläutert. Dabei zeigt:
- Fig. 1: ein Diagramm mit Kurven für die Wirkstofffreisetzung bei drei verschiedenen Zubereitungen;
- Fig. 2: Kurven zur Wirkstofffreisetzung bei Tabletten mit verschieden dicken Filmüberzügen; und
- Fig. 3.1-3.6: Fluorid-Profile von sechs verschiedenen Osteoporosis-Patienten über 24 Stunden, die mit der bekannten Zubereitung sowie mit der erfindungsgemäßen Zubereitung behandelt wurden.

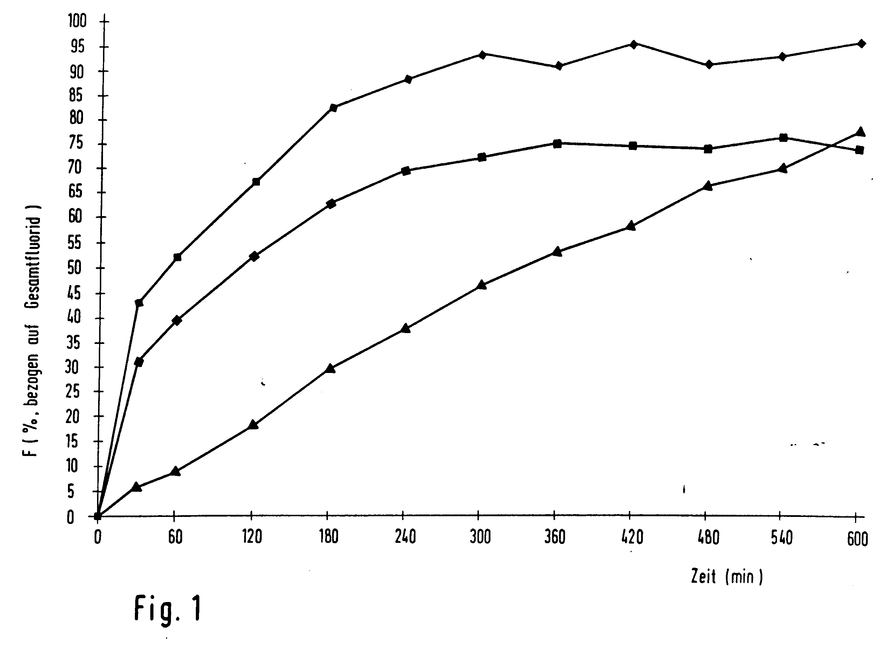

In Fig. 1 sind drei verschiedene Freisetzungskurven für verschiedene Präparate mit Natriumfluorophosphat dargestellt, wobei die in der Senkrechten aufgetragenen Prozentangaben die akkumulierte Freisetzung von Natriumfluorophosphat darstellt und in der Waagerechten die Zeit in Minuten aufgetragen ist.

Die obere Kurve stellt das Freisetzungsprofil für Tabletten gemäß der DE-OS 37 27 616 dar. Die mittlere Kurve ist das Freisetzungsprofil für eine Tablette mit einer Retardmatrix nach der Erfindung, aber ohne den zusätzlichen Überzug, die untere Kurve schließlich stellt ein Freisetzungsprofil für eine Tablette mit dem erfindungsgemäßen dualen Retardsystem dar. Wie leicht zu erkennen ist, wird erst mit der erfinderischen Kombination einer Retardmatrix und eines retardierenden Überzugs eine im wesentlichen lineare Freisetzung von Natriumfluorophosphat erreicht, wobei nach einer Stunde etwa 8 %, nach 6 Stunden etwa 42 % freigesetzt sind. Das dargestellte Freisetzungprofil wurde an einer Tablette gemessen, die 152 mg Natriumfluorophosphat enthält und zur einmal täglichen Applikation gedacht ist (Retardierung 8-10 Stunden). Für eine zweimal tägliche Applikation ist eine 76 mg Formulierung ideal, die ein ähnliches Freisetzungsprofil zeigt (Retardierung 6 bis 8 Stunden).

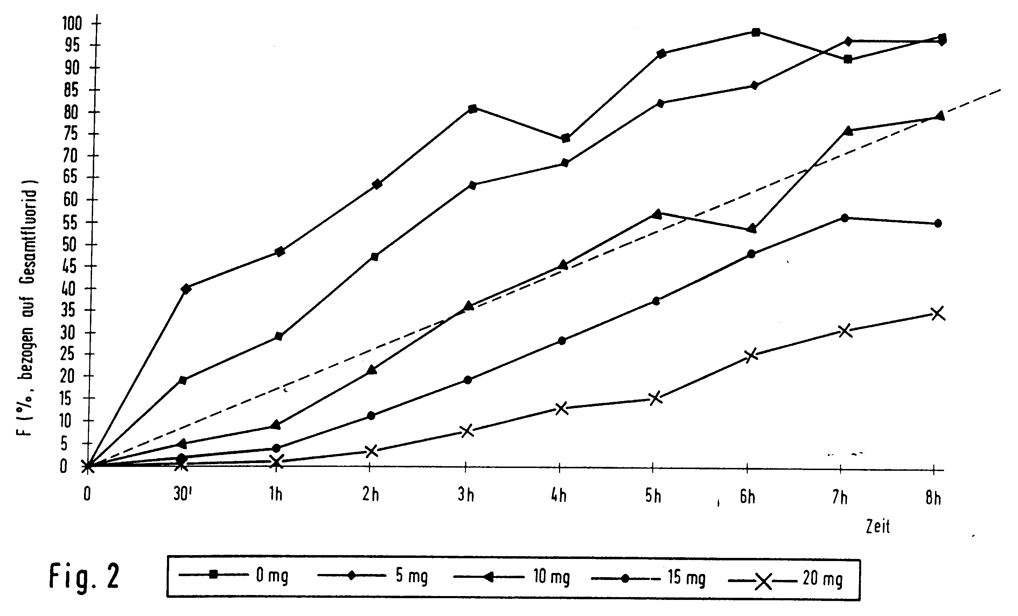

Fig. 2 zeigt Freisetzungsprofile von Tabletten, die in jedem Falle die erfindungsgemäße Retardmatrix enthalten, aber unterschiedlich dicke Filmüberzüge aufweisen, wie angegeben. Die Freisetzungsprofile wurden in künstlich formuliertem Magensaft gemessen, nach Ablauf von zwei Stunden in künstlich hergestellten Darmsaft.

Aus der Darstellung ergibt sich, daß die Geschwindigkeit der Freisetzung im wesentlichen direkt proportional zur Filmdicke ist, wobei (wegen einer im wesentlichen linearen Freisetzung ein Filmüberzug mit 10 mg besonders bevorzugt ist). Im Gegensatz zur Freisetzung bei einer Tablette nach dem Stand der Technik läßt die stärker retardierte und linear ablaufende Freisetzung bei dem dualen Retardsystem nach der Erfindung eine wesentliche Verringerung der gastrointestinalen Nebenwirkungen erwarten.

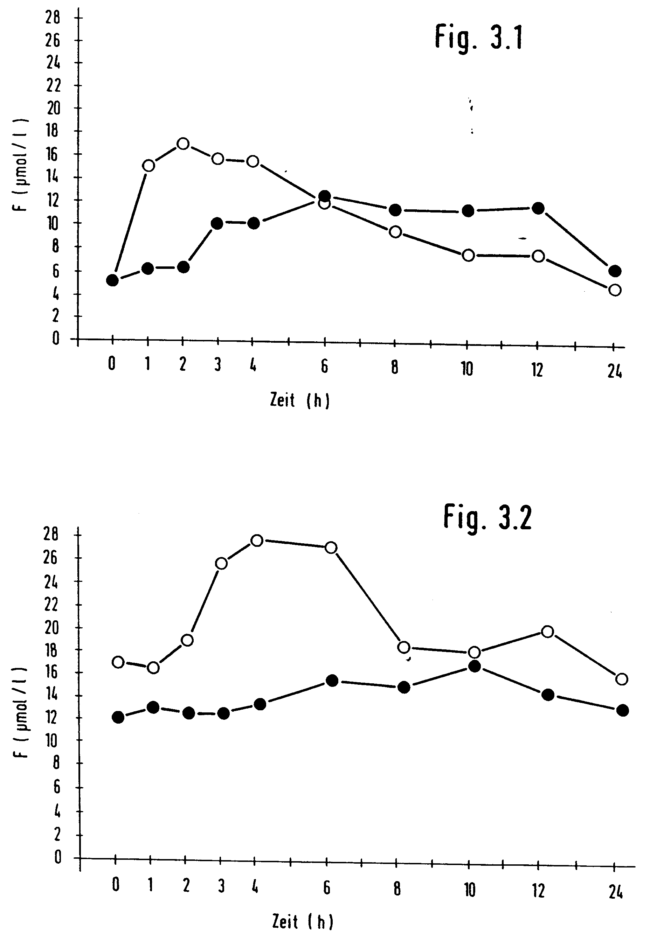

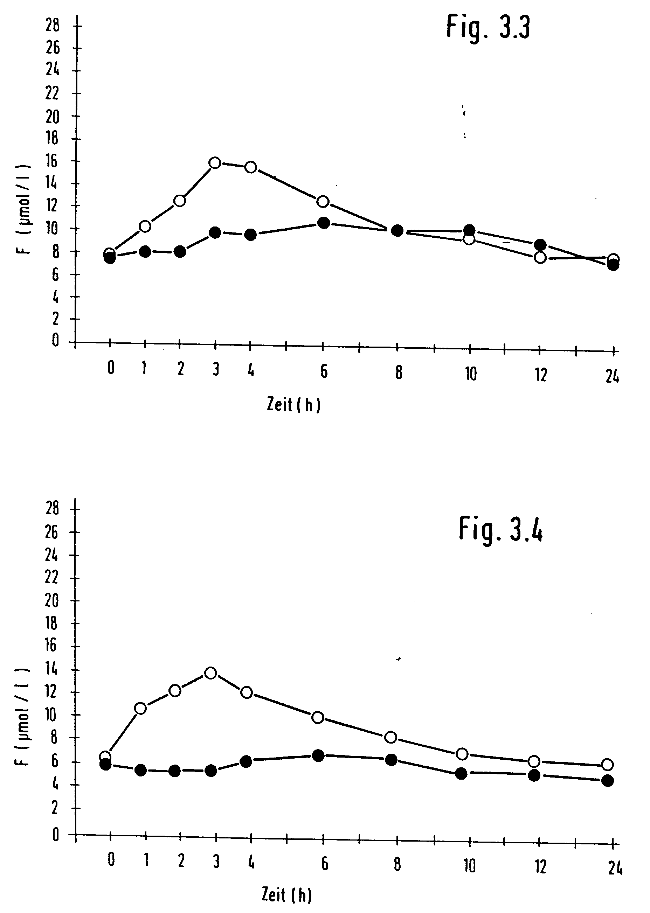

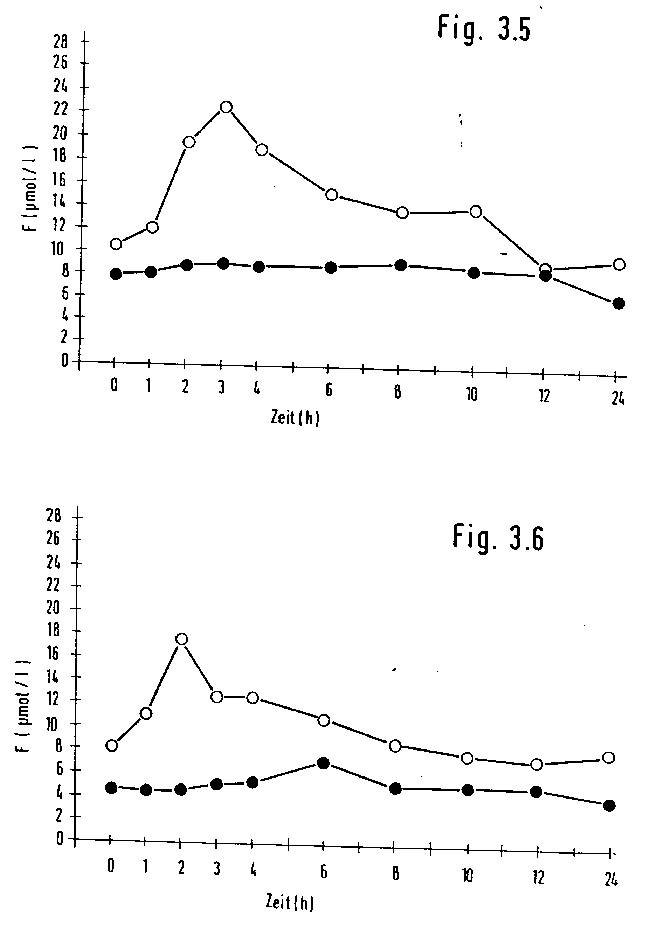

Die dargestellten in-vitro-Untersuchungen wurden durch weitere in-vivo-Untersuchungen untermauert. In den Fig. 3.1 bis 3.6 sind 24-Stunden-Fluoridprofile von sechs weiblichen Osteoporosis-Patienten dargestellt, wobei in der Senkrechten die Fluorid-Konzentration in µmol/l aufgetragen ist und in der Waagerechten die Zeit in Stunde. Die Freisetzungsprofile mit den offenen Kreisen entsprechen der Verabreichung eines Natriumfluorophosphat-Präparats gemäß dem Stand der Technik, während die Freisetzungsprofile mit den gefüllten Kreisen der Verabreichung des erfindungsgemäßen Präparates entsprechen. In allen Fällen ist eine insbesondere in den ersten sechs Stunden deutlich stärker retardierte Freisetzung festzustellen, die wirkungsvoll dazu beiträgt, die gastrointestinalen Nebenwirkungen bei den Patienten zu reduzieren.

### Herstellungsbeispiel

Für eine Charge erfindungsgemäßer Filmtabletten zu 100.000 Stück werden die folgenden Rohstoffe in den folgenden Mengen benötigt:

| | | |
|---|---|---|
| Kern: | Natriumfluorophosphat | 7,980 kg |
| | Eudragit® RSPM | 6,355 kg |
| | Eudragit® RS 30 D | 2,550 kg |
| | Magnesiumstearat | 0,300 kg |
| | Boeson® VP | 0,300 kg |
| | Stearinsäurepulver | 0,400 kg |
| | Aerosil 200 | 0,100 kg |
| Überzug: | Talk | 0,290 kg |
| | Titandioxid | 0,205 kg |
| | Diethylphthalat | 0,034 kg |
| | Eudragit® RS 30 D | 0,570 kg |

Eine lackierte Tablette enthält somit:

| | | |
|---|---|---|
| Kern: | Natriumfluorophosphat | 79,80 mg |
| | Eudragit® RSPM | 63,55 mg |
| | Eudragit® RS 30 D | 25,50 mg |
| | Magnesiumstearat | 3,00 mg |
| | Boeson® VP | 3,00 mg |
| | Stearinsäurepulver | 4,00 mg |
| | Aerosil 200 | 1,00 mg |
| Überzug: | Talk | 2,90 mg |
| | Titandioxid | 2,05 mg |
| | Diethylphthalat | 0,34 mg |
| | Eudragit® RS 30 D | 5,70 mg |

Für die Herstellung können übliche galenische Techniken angewendet werden.

Die in der vorstehenden Beschreibung sowie in den Ansprüchen und den beiliegenden Zeichnungen offenbarten Merkmale der Erfindung können sowohl einzeln als auch in beliebiger Kombination für die Verwirklichung der Erfindung in ihren verschiedenen Ausführungsformen wesentlich sein.

## Patentansprüche

1. Pharmazeutische Zubereitung in Tablettenform für die Fluoridionen-Versorgung zur Behandlung und Verhinderung von Knochensubstanzverlusten, einschließlich Osteoporose und alveolaren Knochenschädigungen, mit einem Gehalt an Natriumfluorophosphat und Mitteln zur Regulierung der Natriumfluorophosphat-Freigabe, dadurch gekennzeichnet, daß die Mittel zur Regulierung der Natriumfluorophosphat-Freigabe ein duales Retardsystem umfassen, das aus einer Retardmatrix im Tablettenkern und einem retardierenden Überzug besteht, wobei sowohl die Retardmatrix als auch der retardierenden Überzug aus Copolymerisaten aus Acryl- und Methacrylsäureestern aufgebaut sind.

2. Pharmazeutische Zubereitung nach Anspruch 1, dadurch gekennzeichnet, daß die Retardmatrix und/oder der retardierende Überzug auf einer wäßrigen Dispersion von Copolymerisaten aus Acryl- und Methacrylsäureestern mit einem geringen Gehalt an quarternären Ammoniumgruppen aufgebaut ist.

3. Pharmazeutische Zubereitung nach einem der vorangehenden Ansprüche, gekennzeichnet durch einen Gehalt an 120 bis 200 mg Natriumfluorophosphat bei einer einmal täglich zu applizieren den Formulierung bzw. durch einen Gehalt von 40 bis 100 mg Natriumfluorophosphat bei einer zweimal täglich zu applizierenden Formulierung.

## Claims

1. A pharmaceutical preparation in tablet form for fluoride ion supply for treatment and prevention of loss of bone substance, including osteoporosis and alveolar bone injury, the preparation containing sodium fluorophosphate and means for regulating the release thereof, characterised in that the means for regulating the release of sodium fluorophosphate comprise a dual retard system made up of a retard matrix at the centre of the tablet and a retarding coating, the retard matrix and the retarding coating both being made up of copolymers of acrylic and methacrylic acid esters.

2. A pharmaceutical preparation according to claim 1, characterised in that the retard matrix and/or the retarding coating are made up of an aqueous dispersion of copolymers of acrylic and methacrylic acid esters with a small content of quaternary ammonium groups.

3. A pharmaceutical preparation according to either of the preceding claims, characterised by a content of 120 to 200 mg sodium fluorophosphate in the case of a formulation for use once a day or a content of 40 to 100 mg sodium fluorophosphate in the case of a formulation for use twice a day.

## Revendications

1. Composition pharmaceutique sous la forme de comprimés pour l'apport d'ions fluorures en vue du traitement et de la prévention des pertes de substances des os, y compris l'ostéoporose et les lésions alvéolaires des os, ladite composition contenant du fluorophosphate de sodium et des agents pour la régulation de la libération du fluorophosphate de sodium, caractérisée par le fait que les agents servant à la régulation de la libération du fluorophosphate de sodium consistent en un système retard double qui consiste lui-même en une gangue retard dans le noyau dit comprimé et un revêtement retardateur, la gangue retard comme le revêtement retardateur étant constitués de copolymères d'esters acryliques et méthacryliques.

2. Composition pharmaceutique selon la revendication 1, caractérisée par le fait que la gangue retard et/ou le revêtement retardateur sont formés à partir d'une dispersion aqueuse de copolymères d'esters acryliques et méthacryliques à faible teneur en groupes ammonium quaternaire.

3. Composition pharmaceutique selon l'une des revendications qui précèdent, caractérisée par une teneur de 120 à 200 mg de fluorophosphate de sodium pour une composition à administrer une fois par jour et par une teneur de 40 à 100 mg de fluorophosphate de sodium pour une composition à administrer deux fois par jour.
